# EUROPEAN PATENT SPECIFICATION

(11) **EP 3 975 824 B1**
(45) Date of publication and mention of the grant of the patent: **07.10.2026**
(21) Application number: 20812566.6
(22) Date of filing: 29.05.2020
(51) Int. Cl.: A61B 3/16, B81B 3/00, G02B 3/00, G02C 9/00, G16H 50/30

(54) **INTRAOCULAR PRESSURE MONITORING CONTACT LENS**
KONTAKTLINSE ZUR ÜBERWACHUNG DES INTRAOKULARDRUCKS
LENTILLE DE CONTACT À SURVEILLANCE DE PRESSION INTRAOCULAIRE

(30) Priority: 31.05.2019 US 201962855132 P
(43) Date of publication of application: 06.04.2022
(73) Proprietor: Queen's University At Kingston, Kingston, Ontario K7L 3N6 (CA); Kingston Health Sciences Centre, Kingston, Ontario K7L 2V7 (CA)
(72) Inventor: CAMPIGOTTO, Angelica, Kingston, Ontario K7L 2E5 (CA); LAI, Yong Jun, Kingston, Ontario K7L 4P9 (CA); CAMPBELL, Robert J., Kingston, Ontario K7L 4B4 (CA); LEAHY, Stephane, Nepean, Ontario K2H 7E5 (CA)
(74) Representative: Sonnenberg Harrison Partnerschaft mbB
(86) International application number: PCT/CA2020/050734
(87) International publication number: WO 2020/237385

(56) References cited:
- EP-B1- 3 082 570
- CA-A1- 2 961 142
- CN-A- 105 919 551
- CN-A- 105 962 887
- CN-A- 109 288 619
- US-A1- 2013 055 819
- US-A1- 2019 076 021
- US-B2- 10 213 140
- AN HONGBIN ET AL: "Microfluidic contact lenses for continuous non-invasive intraocular pressure monitoring", SPIE PROCEEDINGS; [PROCEEDINGS OF SPIE ISSN 0277-786X], SPIE, US, vol. 11053, 7 March 2019 (2019-03-07), pages 110532E - 110532E, XP060116163, ISBN: 978-1-5106-3673-6, DOI: 10.1117/12.2511909
- HONGBIN AN ET AL: "A method of manufacturing microfluidic contact lenses by using irreversible bonding and thermoforming", JOURNAL OF MICROMECHANICS AND MICROENGINEERING, INSTITUTE OF PHYSICS PUBLISHING, BRISTOL, GB, vol. 28, no. 10, 11 July 2018 (2018-07-11), pages 105008, XP020330833, ISSN: 0960-1317, [retrieved on 20180711], DOI: 10.1088/1361-6439/AACEB7

## Description

### Field

This invention relates to non-invasive wearable devices for monitoring and measuring intraocular pressure.

### Background

Glaucoma is an optic neuropathy in which retinal ganglion cell death occurs leading to progressive vision loss. The disease is the second leading cause of vision blindness in the world. The most important modifiable risk factor for glaucoma is intraocular pressure (IOP); careful monitoring of IOP is a major factor in monitoring the progression of the disease and disease management. IOP levels result from a complex interplay of factors influencing the flow of the aqueous humor within the eye. The aqueous humor brings nutrients to the eye and is central to maintaining eye shape for proper light refraction. In healthy eyes, the aqueous humor drains out of the eye via the trabecular meshwork; however, in individuals with glaucoma there is often increased resistance to outflow and consequently elevated IOP. The IOP has been determined to fluctuate throughout the day based on diet, exercise, water intake, and more; with higher levels found when patients are in the supine position and peak IOP in the early mornings.

Goldmann applanation tonometry (GAT) is the current standard for monitoring the IOP. This procedure uses a device that measures the force required to flatten a specified area of the cornea. GAT requires local anesthesia and a trained eye care professional to perform the test. As a result, patients with glaucoma generally have their IOP measured in a clinic setting, in an upright position, every few months and only within typical office hours. However, IOP levels have been found to vary as much as 10 mmHg or more in a single day. More frequent measuring of IOP is therefore needed.

To address this need various approaches have been proposed, some of which are implanted in the eye and therefore invasive and undesirable. Recently, a continuous monitoring contact lens system, Triggerfish^{®} (Sensimed SA, Lauzanne, Switzerland), has been approved for market. This device is able to continuously monitor the IOP using a contact lens on the patient's eye for up to 24 hours using a flexible adhesive antenna worn around the eye that wirelessly receives a signal from the contact lens, connected via a wire to a dedicated electronic reading and data storage device. Drawbacks of the system are discomfort to the patient who must wear the antenna and carry the electronic device, and significant cost.

Overall, the prior approaches are either invasive or require specialized electronic equipment to obtain the measurement data, which reduce the patient's comfort, and increase the complexity and cost of clinical implementation.

HONGBIN AN ET AL: "Microfluidic contact lenses for continuous non-invasive intraocular pressure monitoring", SPIE PROCEEDINGS; 7 March 2019 (2019-03-07), pages 110532E-110532E, DOI: 10.1117/12.2511909 discloses a device and a method for monitoring intraocular pressure of an eye.

HONGBIN AN ET AL: "A method of manufacturing microfluidic contact lenses by using irreversible bonding and thermoforming", JOURNAL OF MICROMECHANICS AND MICROENGINEERING, INSTITUTE OF PHYSICS PUBLISHING, BRISTOL, GB, vol. 28, no. 10, 11 July 2018 page 105008, DOI: 10.1088/1361-6439/AACEB7 discloses a device and a method for monitoring intraocular pressure of an eye.

### Summary

One aspect of the invention relates to a device for monitoring intraocular pressure (IOP) of an eye of a subject according to claim 1, comprising: a flexible lens consisting of one or more hydrophilic oxygen-permeable polymer that is adapted to fit on the eye and to change curvature in response to a change in curvature of the eye; a microchannel disposed in or on the lens, the microchannel having two or more ends, wherein at least two ends are open to a surrounding environment of the lens; an indicator solution disposed in a portion of the microchannel with endpoints of the indicator solution at a position between the two or more open ends of the microchannel; wherein the microchannel exhibits a change in volume in response to a change in curvature of the lens; wherein the change in volume of the microchannel results in a change in the position of the endpoints of the indicator solution between the two or more open ends of the microchannel; wherein the change in position of the endpoints of the the indicator solution in the microchannel is indicative of a change in IOP.

In one embodiment, the microchannel has first and second ends, wherein the first end and the second end are open to a surrounding environment of the lens.

In one embodiment, at least one reference marker disposed on the lens; wherein the change in position of the endpoints of the indicator solution in the microchannel is determined relative to the at least one reference marker.

In one embodiment, the at least one reference marker and the indicator solution are visible in digital images of the device.

Embodiments may comprise a mesh, porous plug, or valve disposed in the at least one open end of the microchannel.

In various embodiments, the microchannel is configured in a convoluted, zig-zag, coiled, spiral, and/or branched shape along a substantial portion of its length. In one embodiment the microchannel comprises a first section configured linearly or in a smooth arc and a second section configured in a convoluted, zig-zag, , coiled, spiral, and/or branched shape along a substantial portion of its length. In one embodiment the indicator solution is disposed in the first section of the microchannel.

The microchannel may be configured to occupy a substantial portion of a surface area of the lens. The microchannel may be configured to occupy an outer region of the lens.

In various embodiments, the indicator solution comprises oil or a water-based solution with a naturally-occurring or added pigment, colour additive, or dye.

Another aspect of the invention relates to a method according to claim 7 for measuring and/or monitoring intraocular pressure (IOP) of an eye of a subject, comprising: fitting a flexible lens on the eye, the flexible lens consisting of one or more hydrophilic oxygen-permeable polymer, the flexible lens being adapted to change curvature in response to a change in curvature of the eye; wherein the flexible lens comprises a microchannel, the microchannel having at least two ends that are open to a surrounding environment of the lens, wherein the microchannel exhibits a change in volume in response to a change in curvature of the lens, and an indicator solution disposed in a portion of the microchannel with endpoints of the indicator solution at a position between the two or more open ends of the microchannel; detecting a change in position of the endpoints of the indicator solution in the microchannel; and correlating the change in position of the endpoints of the indicator solution with a change in IOP.

In one embodiment, the method comprises capturing two or more digital images of the lens at two or more instants in time, respectively; and detecting the change in position of the endpoints of the indicator solution in the microchannel as a difference between the two or more digital images.

In one embodiment, the change in position of the endpoints of the indicator solution in the microchannel is determined relative to a reference marker on the lens.

In one embodiment, the digital images are captured using a digital camera of a personal computing device. The personal computing device may be a smartphone.

In one embodiment, the digital images are captured using a digital camera adapted to be worn by the subject.

Another aspect of the invention relates to system for measuring and/or monitoring intraocular pressure (IOP) of an eye of a subject according to claim 12, comprising: device for monitoring intraocular pressure as described herein; and at least one camera; wherein the at least one camera is adapted to be worn by the subject and to acquire digital images of the device fitted to the eye of the subject.

In one embodiment, the at least one camera is implemented in a frame that is adapted to be worn by the subject.

In one embodiment, the frame is an eyeglasses frame.

In one embodiment, the system includes a software application that is adapted to control the acquiring of digital images by the at least one camera.

### Brief Description of the Drawings

For a greater understanding of the invention, and to show more clearly how it may be carried into effect, embodiments will be described, by way of example, with reference to the accompanying drawings, wherein:
Fig. 1 is a diagram of an IOP measuring device according to one embodiment.

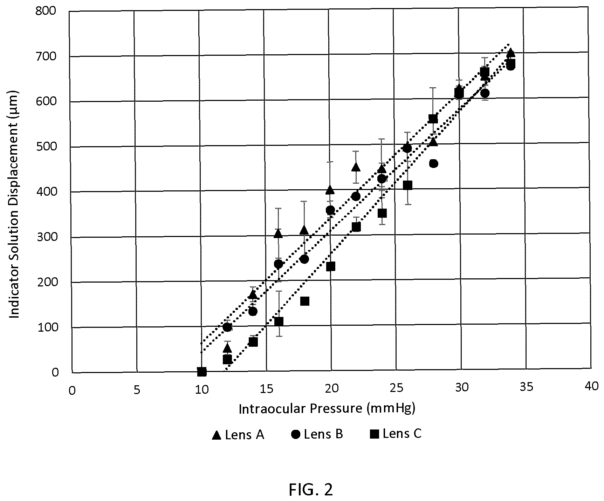

Fig. 2 is a plot showing indicator solution displacement as a function of intraocular pressure for three devices (A, B, C) based on the embodiment of Fig. 1 in an experiment using porcine eyes; each data point is the average of two measurements, error bars show standard deviation.
Fig. 3 is a plot showing indicator solution displacement as a function of intraocular pressure for one device based on the embodiment of Fig. 1 in an experiment using three porcine eyes; each data point is the average of two measurements, error bars show standard deviation.
Figs. 4A and 4B are plots showing indicator solution displacement as a function of fluctuating intraocular pressure for one device based on the embodiment of Fig. 1 in an experiment using a porcine eye.
Figs. 5A, 5B, and 5C are diagrams of IOP measuring devices according to further examples not falling under the scope of the claimed invention for Fig 5A and Fig. 5c and illustrating an embodiment of the claimed invention for Fig. 5B.
Figs. 6A and 6B are plots showing indicator solution displacement as a function of intraocular pressure for the example of Fig. 5A and the embodiment of Fig. 5B, respectively, in experiments using porcine eyes, wherein each data point is the average of two measurements and error bars show standard deviation.
Figs. 7A and 7B are plots showing the effect of central corneal thickness and radius of corneal curvature on the indicator solution displacement as a function of intraocular pressure for the embodiment of Fig. 1, in experiments using porcine eyes.

### Detailed Description of Embodiments

Embodiments described herein address the drawbacks of prior IOP measuring approaches by providing non-invasive and low cost devices that are easily and conveniently interfaced with readily available equipment. Furthermore, the embodiments also address the need to obtain IOP data more frequently, and outside of regular clinic hours. For the embodiments described, measurement data is obtained optically and may be easily acquired using a camera. For example, readily available cameras such as those found on smartphones and tablet computers may be used for data acquisition. By acquiring images over a period of time, a patient's IOP profile may be precisely determined over time.

Since the shape of the eye, i.e., the corneal curvature, changes in response to changes in IOP, contact lens- (or cosmetic lens-) based embodiments were developed that respond to changes in corneal curvature by deforming, in which an amount of deformation can be directly related to a change in IOP. In general, embodiments are based on a flexible contact lens or cosmetic lens, in and/or on which is disposed a microchannel.

As used herein, the terms "contact lens", "cosmetic lens", and "lens" are used interchangeably and are intended to refer to any type of flexible lens that may be fitted to and worn on the eye, such as, for example, a contact lens or a cosmetic lens. A flexible lens may fit substantially only on the cornea, or it may fit on the cornea and extend outwardly from the cornea to also fit over a portion of the conjunctiva and sclera.

According to the embodiments, at least a portion of the microchannel exhibits a change in volume in response to deformation of the contact lens resulting from a change in corneal curvature. The microchannel has two or more ends that are open to the surrounding environment or medium, e.g., the atmosphere (i.e., air). The open ends of the microchannel may be located at an outer edge of the contact lens, or in close proximity to the outer edge. The microchannel is partially filled with an indicator solution that provides a visual indication of the microchannel change in volume, as the indicator solution is displaced along the microchannel in reaction to a volume change. Some embodiments may include a mesh, porous plug, valve, etc., in at least one of the one or more open end to keep the indicator solution from leaking out of the microchannel while allowing air to pass. For example, a one-way valve may use the fluid tension of the indicator solution and the small channel size to prevent back flow of the solution out of the microchannel. The flexible contact lens together with the microchannel design and the indicator solution allow for the device to capture fluctuating corneal curvature by correlating with fluctuation in the internal volume of the channel, according to movement of the indicator solution.

The indicator solution may be a liquid that is typically incompressible or substantially incompressible, to ensure maximum displacement within the microchannel in response to deformation of the contact lens. The indicator solution may be biocompatible and/or isotonic with the eye. Examples include oils (e.g., plant-based oils) and water-based solutions with naturally-occurring or added pigments, colour additives, or dyes. Examples of colour additives include, but are not limited to, those approved for use in medical devices and contact lenses according to the U.S. Code of Federal Regulations Title 21, Part 74, subpart D.

In various embodiments, the microchannel has a dimension and/or shape that is selected such that the change in volume in response to a selected amount of change in corneal curvature causes the indicator solution to be displaced sufficiently within the microchannel so that the displacement is detectable in an image captured by a digital camera. For example, the microchannel may have a substantial length so as to provide the required amount of change in volume. The substantial length may be achieved by providing the microchannel in a convoluted, zig-zag, , coiled, spiral, and/or branched, etc., shape, and/or the microchannel may be configured to occupy a substantial portion of the surface area of the contact lens. In some embodiments, the microchannel occupies the outer region or periphery of the contact lens, so as to avoid, as much as possible, interfering with the vision of the person wearing the device. In some embodiments the microchannel may have a portion that is disposed along the entire periphery or most of the periphery of the contact lens, e.g., the microchannel may be disposed in 270-360° of the outer region of the lens. It will be appreciated from the below examples that the length of the microchannel may vary significantly, depending on the design. In various embodiments, the length of the microchannel may be, for example, about 140 mm to about 280 mm, although other lengths may be used. In some embodiments the microchannel is sufficiently long such that a reservoir for the indicator solution is not needed, resulting in more consistent and reliable response to changes in IOP. The microchannel may have a circular, rounded, rectangular, etc., cross-section, and an approximate diameter of about 60 µm to about 140 µm. In one embodiment the microchannel diameter is approximately 100 µm. In some embodiments there is a widening of the microchannel at the one or more open end. For example, the one or more open end may be widened to about 1 mm.

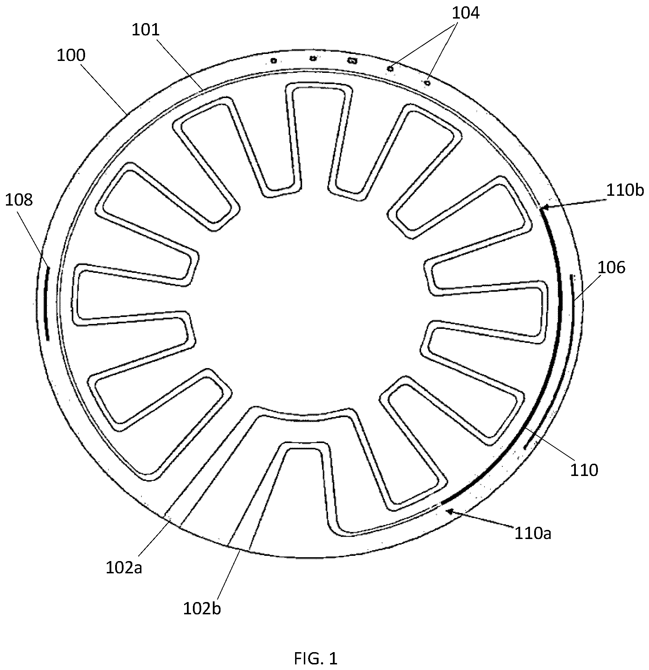

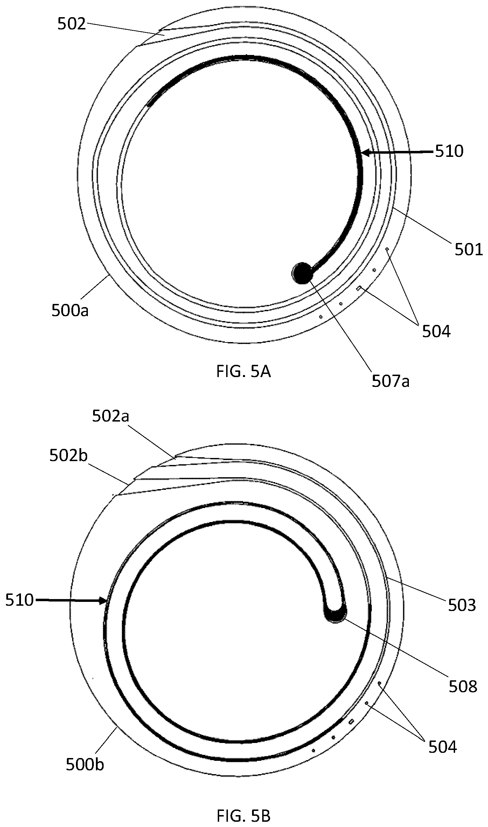

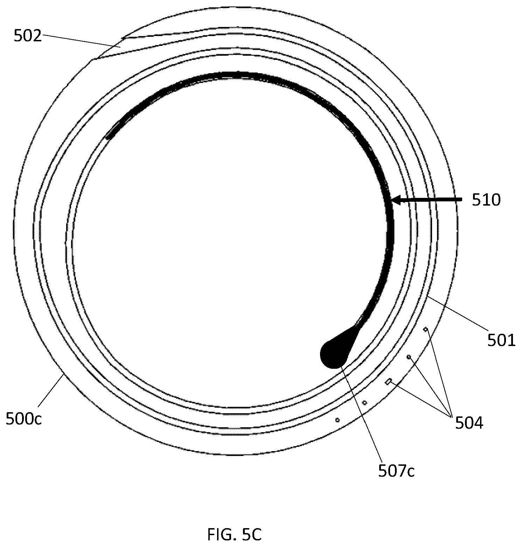

Non-limiting examples of such possible microchannel configurations are shown in Figs. 1, 5A, 5B, and 5C. Of course, other configurations may be implemented to achieve substantially the same or similar results. Regardless of the configuration of the microchannel, at least one end of the microchannel is open, so that it is exposed to the atmosphere. Without wishing to be bound by theory, it is suggested that due to the micro-scale of the microchannel, a sealed microchannel experiences an internal vacuum/pressure build-up that may inhibit or halt the indicator solution movement. Providing the at least two ends open to the atmosphere removes any such forces, and instead a pressure difference between the atmosphere and microchannel facilitates the indicator solution movement.

According to the embodiments, to detect a change in IOP of, e.g., 1 mmHg, the corresponding change in the microchannel volume is large enough to cause the indicator solution to be displaced sufficiently to be detected in a digital image taken by a readily-available camera. For example, the change in position of the indicator solution may be detected in images taken by a camera having a resolution typical of a smartphone camera or other electronic device, or other suitable camera, including, for example, a camera implemented in an eyeglasses frame or other mount that can be worn by the subject. The change in position of the indicator solution within the microchannel may be determined relative to one or more reference markers provided on the contact lens. The reference markers and the indicator solution are visible in the digital images.

Digital images of the contact lens are acquired and analyzed to detect locations of the indicator solution in the microchannel, and these are correlated to actual IOP. The images may be taken using a digital camera of a mobile device such as a smart phone, tablet, or laptop computer, etc., and therefore, the image may be easily taken by the subject without the need for specialized equipment and at any time of the day or night, without a professional's intervention.

An application running on the mobile device used to obtain the digital images, or on a remote device, may include an algorithm that provides image analysis required to determine IOP from the images, and may provide substantially instant feedback about the subject's IOP. For example, a software application as described in U.S. Patent Application Publication No. 2017-0280997-A1 may be used. The application may prompt the subject to obtain images at selected times or according to a selected schedule. In the case of a camera worn by the subject, such as a camera implemented in an eyeglasses frame, the camera may communicate with the device running the software application (e.g., via Bluetooth^{®}), and the application may direct the camera to acquire images automatically, e.g., at selected times or according to a selected schedule. This may include notifying the subject when an image is being acquired, so that the subject's eye is open for proper image acquisition. The application may conduct a preliminary analysis of an acquired image to ensure its suitability (i.e., sufficient quality) for determining IOP, and reject unsuitable images and acquire further images until a suitable image is obtained. The images may be stored on the device, and accumulated for later uploading to a server (e.g., at a doctor's office) at a selected or prescribed time or schedule using a wired or wireless connection. Alternatively, the images may be uploaded substantially immediately as acquired if a suitable connection is available.

Embodiments may be constructed using a flexible polymer material suitable for contact lens applications. According to the claimed invention, the material is chosen so that a contact lens is hydrophilic for proper interaction with the tear film. A contact lens prepared from a hydrophobic material may be subjected to suitable surface treatment to achieve hydrophilicity. For extended wear the contact lens may be oxygen-permeable, e.g., about 6 to about 150 Dk/t.

The material may comprise silicone (e.g., polydimethyl siloxane (PDMS)). Other materials such as polymethyl methacrylate (PMMA) may be used in some embodiments, although such material is less flexible. The material may comprise a hydroxyethylmethacrylate (HEMA), which may be in the form of a hydrogel such as a polyHEMA, or a HEMA cross-linked with ethylene dimethacrylate (EDMA) or ethylene glycol dimethacrylate (EGDMA). In some embodiments combinations of two or more such materials may be used, such as a silicone-hydrogel, a HEMA hydrogel, or a HEMA/MAA. Silicone hydrogel contact lenses have a high oxygen permeability, typically >100 Dk. In some embodiments a readily available (i.e., off-the-shelf) lens may be used, which may be machined or modified to obtain the desired microchannel. Embodiments may be constructed by one or more of: molding a lens including a microchannel; machining a microchannel into a lens; and applying a microchannel to a lens. Molding may include preparing a 3D mold with the microchannel features using a high speed micromilling machine.

For example, a silicone polymer is hydrophobic, despite its high oxygen permeability. Therefore, silicone is not ideal as a homopolymer contact lens material. However, a copolymer of silicone with a hydrophilic (highly polar) monomer can solve this problem. Copolymerization may also be used to enhance physical properties through the cross-linking of polymer chains by adding molecular weight to the chain. In some cases, adding a soft polymer can reduce the modulus of a particularly rigid material (often a silicone-based material). Hydrophobic co-monomers add oxygen permeability to materials that require improvement in this property, and silicone-based monomers are often used for this purpose.

The invention is further described by way of the following non-limiting Examples.

### Example 1

An embodiment of the device is shown diagrammatically in Fig. 1. As shown in Fig. 1, a flexible contact lens 100 approximately 250 µm thick was made from polydimethylsiloxane (PDMS). The contact lens was manufactured with an embedded microchannel 101 with a cross section of approximately 100 µm X 100 µm, with a cover layer (not shown) that covered the base and sealed the microchannel. The microchannel had an inlet 102a and an outlet 102b which were tapered to about 1 mm at the openings to allow for injecting the indicator solution, shown darkened at 110, into the microchannel. The microchannel had two sections, an inner "zig-zag" or "crenellated" section with an approximate diameter of 100 µm and an outer section without sharp curves or changes in direction (e.g., substantially linear, or curvature with constant or varying radius such as a smooth arc or other shape), and approximate diameter of 80 µm close to the outer edge of the lens. The total length of the microchannel was about 260 mm. In this embodiment the indicator solution was disposed in the outer section of the microchannel but not in the inner zig-zag section. This avoids any impediment to movement of the indicator solution in the microchannel that may be presented by sharp bends and changes in direction in the microchannel. Various reference markers 104, 106, 108 were embedded into the contact lens. In this embodiment it can be seen how the position of the indicator solution 110 in the microchannel 101 can be determined according endpoints 110a and 110b of the indicator solution relative to the reference marker 106. It will be appreciated that other shapes, patterns, etc., of the inner section of the microchannel, other than a zig-zag pattern, may be used in order to achieve a selected total length of the microchannel (e.g., about 140 mm to about 300 mm) that provides detectable movement of the indicator solution in response to a change in IOP.

The contact lens was manufactured using a casting process. Mold components were manufactured from aluminum using a Model 363-S micro-milling machine (Microlution, Inc.), with a feed rate of 8,000 rpm. PDMS was used as the main material for the contact lens device for its flexibility and oxygen permeability, and is a known FDA approved biocompatible material, already used to manufacture soft contact lenses. Elasticity of the PDMS can be modified based on the amounts (i.e., ratio) of pre-polymer and curing agent used. For example, whereas a ratio of 10:1 is common, the ratio was optimized herein to have as low an elastic modulus as possible without the presence of the adhesive properties that can occur. For this embodiment, the ratio was optimized at 15:1. First, PDMS pre-polymer and curing agent were mixed at a ratio of 15:1 and poured into the mold. The mold was placed in a vacuum chamber for 40 minutes for degassing, until substantially all air bubbles disappeared. The mold was placed in an oven at 65°C for 40 minutes and then was cooled to room temperature, and then the contact lens was released from the mold. A thin, approximately 100 µm, cover layer was made using a similar casting process, with a smooth mold. The two components (base and cover layer) were surface treated using a Corona Treater (Electro-Technic Products Inc., Chicago, IL, USA), then bonded together for a total lens thickness of 250 um. For the indicator solution, dyed biocompatible avocado oil was injected into one of the open ends of the microchannel. In a realization not according to the claimed subject-matter, one of the ends of the microchannel was then sealed with PDMS while the other end was left open to the atmosphere. Avocado oil was selected as the indicator solution due to its heavy molecule structure, making it unable to evaporate from the PDMS while having sufficient colour to visually track its movement throughout the microchannel. The indicator solution was positioned far enough away from the open end to avoid the possibility of it leaking out of the microchannel.

### Example 2

Performance of an embodiment as shown in Fig. 1 and as described in Example 1 was evaluated in a series of experiments using fresh enucleated porcine eyes. Porcine eyes were chosen for their similarities in shape and mechanical properties to human eyes. Testing was performed with a slow and constant stress applied to an eye while a contact lens directly measuring the strain. To mitigate any unwanted affects from the eye drying out and to add a tear layer to the system, water was dripped onto the porcine eye throughout the experiments.

For the tests, a porcine eye was placed on an eye holder with a loose fit to allow for unrestricted expansion/contraction of the eye, to avoid any unwanted affect on the IOP. Tubing connected to a syringe pump was inserted into the sclera of the eye. The syringe pump, filled with water, was used to manually simulate changes in IOP at a controlled rate between 10 to 34 mmHg. Real time IOP was monitored with a Miller Micro-Tip Pressure Catheter Transducer threaded into an opposite side of the sclera. The contact lens was placed onto the centre of the cornea and cameras (Duratool Model S02 USB Digital Microscope) recorded the top view and side view of the eye throughout the experiment. The side view of the eye was used to ensure the contact lens had proper connection and placement and to confirm that deformation of the eye curvature did occur, while the top view recorded the indicator solution movement.

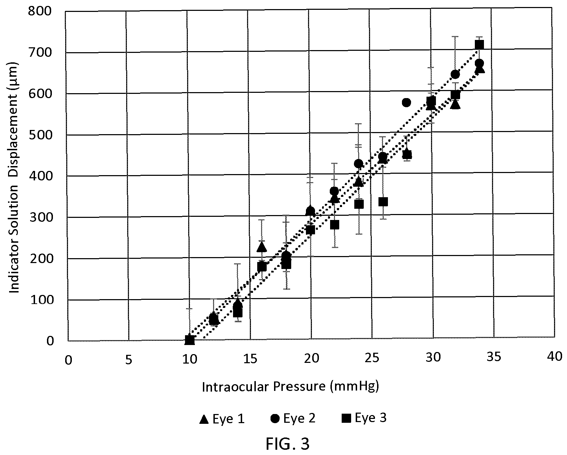

To evaluate reliability, responsiveness, and inter-device agreement, three contact lens devices were each tested two times on a single eye. At baseline and at every 2 mmHg step increase in IOP from 10 mmHg to 34 mmHg, an image of the contact lens device was captured. Absolute displacement of the indicator solution was calculated by comparing to a reference marker (e.g., such as 104, 106, 108 in Fig. 1). For all three devices and trials, the indicator solution showed a similar linear relationship between solution displacement and IOP (Fig. 3). The average indicator solution displacement between the trials was 28.5 µm/mmHg with a standard deviation of 2.4. The small differences between devices in indicator solution movement may have resulted from inconsistencies introduced in fabrication, the effects of positioning of the lens on the eye or small errors introduced by the micro-catheter pressure transducer used to determine the a standard IOP measure.

To evaluate the ability of the embodiment to reliably detect the IOP of different eyes, a single device was tested on three eyes. The three tested eyes had different corneal size, shape and thickness, with related differences in biomechanical properties, which represent typical variations from eye to eye. Similar to the above experiment, the indicator solution was analysed at baseline and at every 2 mmHg increase in IOP from 10 mmHg to 34 mmHg. For all three eyes the indicator solution movement showed a similar linear relationship with increasing IOP at slopes of 26.4, 29.3, and 28 µm/mmHg for eye 1, 2 and 3, respectively (Fig. 3), resulting in an average indicator solution movement between trials of 27.9 µm/mmHg with a standard deviation of 1.5. The consistent results demonstrate the robustness of the IOP device to physiological variations, i.e., the device provides reliable IOP measurement for different eyes. Based on the experimental results, it may be concluded that the flexibility of the contact lens device allows it to overcome variations in physiology between porcine eyes. It is expected that similar performance would be obtained for human eyes.

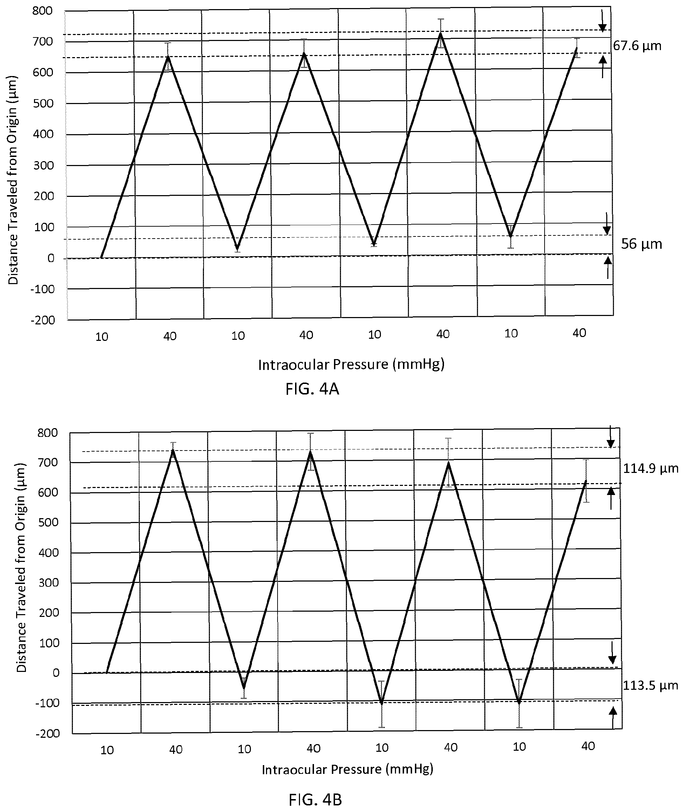

To accurately track fluctuations in IOP over an extended period, such as a day, the device must allow indicator solution travel in an anterograde direction with increases in IOP and a retrograde direction with decreases in IOP, over multiple cycles, with high repeatability. Therefore, to evaluate this capability, IOP was cycled between 10 mmHg and 40 mmHg for a total of four cycles and the indicator solution position was determined from camera images. There were noticeable differences in results depending on the tracking point of the indicator solution used. The outer tracking point (110a in Fig. 1) yielded more consistent results compared to the inner tracking point (110b in Fig. 1); see Figs. 4A and 4B, respectively. For the outer tracking point there was a maximum variance in the indicator solution position of 56.7 µm when the pressure was 10 mmHg and 67.6 µm when the pressure was 40 mmHg. The indicator solution returned 92% of the way back to the original position for the outer tracking point and 85% for the inner tracking point. It is believed that the difference is due to microchannel retention of the indicator solution; for example, retention of the indicator solution on the sidewalls of the microchannel reduces the effective volume of the indicator solution. As fluid retention within the microchannel increases -with multiple trials- the decrease in the initial volume of the indicator solution has a differential effect on the tracking points.

### Example 3

An alternative example is shown in Fig. 5A. In the example of Fig. 5A the contact lens device 500a has a microchannel 501 in a single spiral configuration with an approximate diameter of 100 µm and total length of about 180 mm, and an open end 502 that was tapered to about 1 mm. Reference markers 504 are provided along an edge of the device. An indicator solution, shown darkened at 510, is disposed in a portion of the microchannel. Optionally, a reservoir 507a for the indicator solution may be disposed at the end of the microchannel 501.

The embodiment of Fig. 5B is similar to the example shown in Fig. 5A, except that the microchannel 503 is in a double spiral configuration, such that both ends 502a, 502b are open to the atmosphere. The microchannel has an approximate diameter of 100 µm and total length of about 190 mm, and open ends 502 that are tapered to about 1 mm. An indicator solution 510 is disposed in a portion of the microchannel. Optionally, a widening of the microchannel may be provided at a point 508 where the two microchannel spirals are connected.

The example of Fig. 5A and the embodiment of Fig. 5B were manufactured in PDMS pre-polymer and curing agent at a ratio of 15:1 using micro-machined aluminum molds in the same manner as described in Example 1.

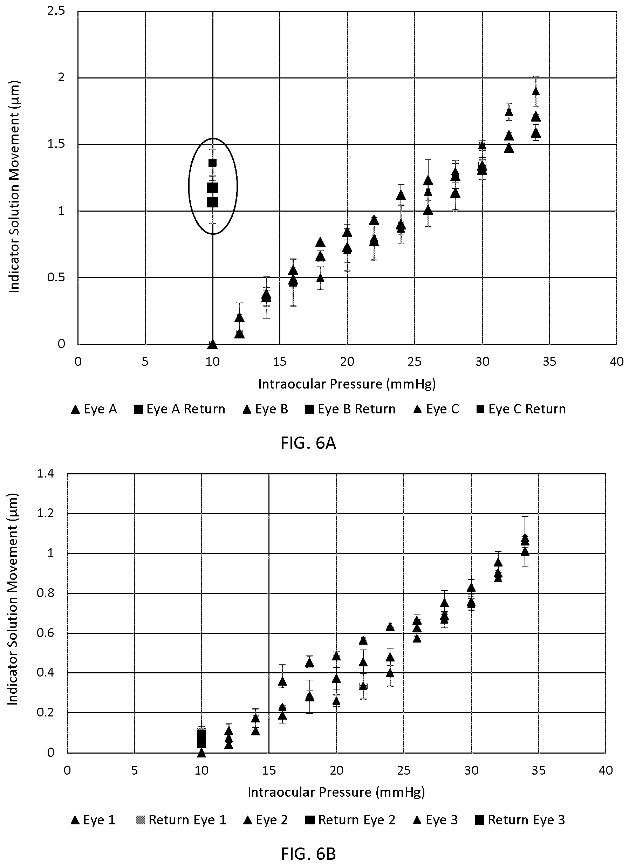

Figs. 6A and 6B show results of preliminary tests of the example of Fig. 5A and the embodiment of Fig. 5B, respectively. The methods were as described in Example 2. One device of each example was fitted to three different porcine eyes, and two trials were completed for each eye. For each trial, images of the device were obtained for 2 mmHg increments in IOP from 10 mmHg to 34 mmHg, and then back to 10 mmHg. The error bars in the plots of Figs. 6A and 6B show the standard deviation for the two trials that were conducted from a single contact lens, on a single eye at each IOP increment.

From Figs. 6A and 6B it can be seen that the single spiral example of Fig. 5A was more sensitive, i.e., there was greater movement of the indicator solution per increment in IOP. From the average slopes the performance was determined to be about 72 µm/mmHg for the single-spiral example, and about 44 µm/mmHg for the double-spiral example. However, in the single-spiral example the indicator solution did not return to the starting position when the IOP was lowered back to 10 mmHg (see the points circled in Fig. 6A). As a result, the overall return rate of this example was 30.7%. In contrast, in the double-spiral example the indicator solution did return to the starting position when the IOP was lowered back to 10 mmHg. As a result, the overall return rate of this example was 98%.

A further example is shown in Fig. 5C. In this example, which is similar to Fig. 5A, the contact lens device 500c has a microchannel 501 in a single spiral configuration with an approximate diameter of 100 µm and total length of about 180 mm, and with an open end 502 that was tapered to about 1 mm. Reference markers 504 are provided along an edge of the device. An indicator solution 510 is disposed in a portion of the microchannel. In this example, a reservoir 507c for the indicator solution is disposed at the end of the microchannel 501. Unlike the optional reservoir 507a of Fig. 5A, in this example there is a smooth curved or linear transition (i.e., without corners or angles) from the reservoir 507c to the microchannel 501 to facilitate movement of the indicator solution.

### Example 4

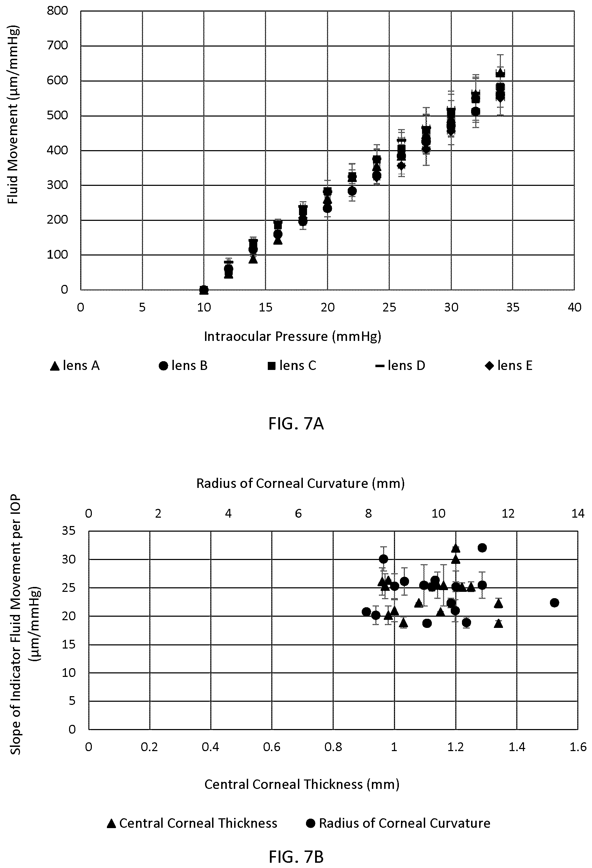

To evaluate the effect of the physiological variations in the central corneal thickness and the radius of corneal curvature on the association between indicator solution to displacement and intraocular pressure, five devices (based on the embodiment of Fig. 1) were tested on 16 porcine eyes, with one to three devices tested on each eye and two trials completed per device. The central corneal thickness and radius of corneal curvature were measured using ultrasound bio-microscopy (UBM). Similar to the above experiments, the indicator solution position was analysed at baseline and after every 2 mmHg increase in IOP from 10 mmHg to 34 mmHg. Fig. 7A shows that all five devices performed similarly, with strong linear slopes of 25.54, 22.86, 24.04, 24.61, and 22.08 µm/mmHg. To further examine the impact of physiological variations, the slopes of the indicator solution movement as a function of the IOP were plotted against the measured central corneal thickness and radius of corneal curvature for the 16 eyes. Fig. 7B shows that there was no correlation between the physiological variations measured and the movement of the indicator solution as a result of the IOP fluctuations.

### Equivalents

Those of ordinary skill in the art will recognize, or be able to ascertain through routine experimentation, equivalents to the embodiments described herein. Such equivalents are within the scope of the invention and are covered by the appended claims.

## Claims

1. A device for monitoring intraocular pressure (IOP) of an eye of a subject, comprising:
a flexible lens (100, 500a, 500b, 500c) consisting of one or more hydrophilic oxygen-permeable polymer that is adapted to fit on the eye and to change curvature in response to a change in curvature of the eye;
a microchannel (101, 501, 503) disposed in or on the lens, the microchannel having two or more ends, wherein at least two ends are open to a surrounding environment of the lens (100, 500a, 500b, 500c);
an indicator solution (110, 510) disposed in a portion of the microchannel with endpoints (110a, 110b) of the indicator solution at a position between the two or more open ends of the microchannel (101, 501, 503);
wherein the microchannel exhibits a change in volume in response to a change in curvature of the lens (100, 500a, 500b, 500c);
wherein the change in volume of the microchannel results (101, 501, 503) in a change in the position of the endpoints (110a, 110b) of the indicator solution (110, 510) between the two or more open ends of the microchannel (101, 501, 503);
wherein the change in position of the endpoints (110a, 110b) of the indicator solution (110, 510) in the microchannel (101, 501, 503) is indicative of a change in IOP.

2. The device of claim 1, comprising:
at least one reference marker (106, 104, 108, 504) disposed on the lens (100, 500a, 500b, 500c);
wherein the change in position of the endpoints (110a, 110b) of the indicator solution (110, 510) in the microchannel (101, 501, 503) is determined relative to the at least one reference marker (106, 104, 108, 504).

3. The device of claim 2, wherein the at least one reference marker (106, 104, 108, 504) and the indicator solution (110, 510) are visible in digital images of the device.

4. The device of claim 1, comprising a mesh, a porous plug, or a valve disposed in the at least one open end of the microchannel (101, 501, 503).

5. The device of claim 1, wherein the microchannel (101, 501, 503) comprises a first section configured linearly or in a smooth arc and a second section configured in a convoluted, zig-zag, coiled, spiral, and/or branched shape along a substantial portion of its length;
optionally wherein the microchannel (101, 501, 503) is configured to occupy an outer region of the lens.

6. The device of claim 5, wherein the indicator solution (110, 510) is disposed in the first section of the microchannel (101, 501, 503);
optionally wherein the indicator solution (110, 510) comprises oil or a water-based solution with a naturally-occurring or added pigment, colour additive, or dye.

7. A method for measuring and/or monitoring intraocular pressure (IOP) of an eye of a subject, comprising:
fitting a flexible lens (100, 500a, 500b, 500c) on the eye, the flexible lens consisting of one or more hydrophilic oxygen-permeable polymer, the flexible lens being adapted to change curvature in response to a change in curvature of the eye;
wherein the flexible lens comprises a microchannel (101, 501, 503), the microchannel having at least two ends that are open to a surrounding environment of the lens (100, 500a, 500b, 500c), wherein the microchannel exhibits a change in volume in response to a change in curvature of the lens (100, 500a, 500b, 500c), and an indicator solution (110, 510) disposed in a portion of the microchannel (101, 501, 503) with endpoints (110a, 110b) of the indicator solution at a position between the two or more open ends of the microchannel;
detecting a change in position of the endpoints (110a, 110b) of the indicator solution (110, 510) in the microchannel (101, 501, 503); and
correlating the change in position of the endpoints (110a, 110b) of the indicator solution (110, 510) with a change in IOP.

8. The method of claim 7, wherein the microchannel (101, 501, 503) comprises a first section configured linearly or in a smooth arc and a second section configured in a convoluted, zig-zag, coiled, spiral, and/or branched shape along a substantial portion of its length;
optionally wherein the microchannel (101, 501, 503) is configured to occupy an outer region of the lens.

9. The method of claim 8, wherein the indicator solution (110, 510) is disposed in the first section of the microchannel (101, 501, 503).

10. The method of claim 7, comprising capturing two or more digital images of the lens at two or more instants in time, respectively; and
detecting the change in position of the endpoints (110a, 110b) of the indicator solution (110, 510) in the microchannel (101, 501, 503) as a difference between the two or more digital images;
optionally wherein the digital images are captured using a digital camera of a personal computing device or using a digital camera adapted to be worn by the subject;
optionally wherein the personal computing device is a smartphone.

11. The method of claim 7, wherein the flexible lens (100, 500a, 500b, 500c) further comprises at least one reference marker (106, 104, 108, 504);
wherein the change in position of the endpoints (110a, 110b) of the indicator solution (110, 510) in the microchannel (101, 501, 503) is determined relative to the reference marker (106, 104, 108, 504).

12. A system for measuring and/or monitoring intraocular pressure (IOP) of an eye of a subject, comprising:
the device of claim 1; and
at least one camera;
wherein the at least one camera is adapted to be worn by the subject and to acquire digital images of the device fitted to the eye of the subject;
optionally wherein the at least one camera is implemented in a frame that is adapted to be worn by the subject;
optionally wherein the frame is an eyeglasses frame.

13. The system of claim 12, further comprising a software application that is adapted to control acquiring of digital images by the at least one camera.

## Patentansprüche

1. Vorrichtung zum Überwachen des Augeninnendrucks (IOP) eines Auges eines Subjekts, umfassend:
eine flexible Linse (100, 500a, 500b, 500c), bestehend aus einem oder mehreren hydrophilen sauerstoffdurchlässigen Polymeren, die angepasst sind, um auf dem Auge angebracht zu werden und die Krümmung als Reaktion auf eine Änderung der Krümmung des Auges zu ändern;
einen Mikrokanal (101, 501, 503), der in oder auf der Linse angeordnet ist, der Mikrokanal aufweisend zwei oder mehr Enden, wobei mindestens zwei Enden zu einer Umgebung der Linse (100, 500a, 500b, 500c) offen sind;
eine Indikatorlösung (110, 510), die in einem Abschnitt des Mikrokanals mit Endpunkten (110a, 110b) der Indikatorlösung an einer Position zwischen den zwei oder mehr offenen Enden des Mikrokanals (101, 501, 503) angeordnet ist;
der Mikrokanal aufweisend eine Änderung des Volumens als Reaktion auf eine Änderung der Krümmung der Linse (100, 500a, 500b, 500c);
die Änderung des Volumens des Mikrokanals (101, 501, 503) führend zu einer Änderung der Position der Endpunkte (110a, 110b) der Indikatorlösung (110, 510) zwischen den zwei oder mehr offenen Enden des Mikrokanals (101, 501, 503);
die Änderung der Position der Endpunkte (110a, 110b) der Indikatorlösung (110, 510) anzeigend in dem Mikrokanal (101, 501, 503) eine Änderung des IOP.

2. Vorrichtung nach Anspruch 1, umfassend:
mindestens einen Referenzmarker (106, 104, 108, 504), der auf der Linse (100, 500a, 500b, 500c) angeordnet ist;
wobei die Änderung der Position der Endpunkte (110a, 110b) der Indikatorlösung (110, 510) in dem Mikrokanal (101, 501, 503) relativ zu dem mindestens einen Referenzmarker (106, 104, 108, 504) bestimmt wird.

3. Vorrichtung nach Anspruch 2, wobei der mindestens eine Referenzmarker (106, 104, 108, 504) und die Indikatorlösung (110, 510) in digitalen Bildern der Vorrichtung sichtbar sind.

4. Vorrichtung nach Anspruch 1, umfassend ein Netz, einen porösen Stopfen oder ein Ventil, das in dem mindestens einen offenen Ende des Mikrokanals (101, 501, 503) angeordnet ist.

5. Vorrichtung nach Anspruch 1, der Mikrokanal (101, 501, 503) umfassend einen ersten Abschnitt, der linear oder in einem glatten Bogen eingerichtet ist, und einen zweiten Abschnitt, der in einer gewundenen, Zickzack-, gewickelten, spiralförmigen und/oder verzweigten Form entlang eines wesentlichen Abschnitts seiner Länge eingerichtet ist;
optional ist der Mikrokanal (101, 501, 503) eingerichtet, um einen äußeren Bereich der Linse einzunehmen.

6. Vorrichtung nach Anspruch 5, wobei die Indikatorlösung (110, 510) in dem ersten Abschnitt des Mikrokanals (101, 501, 503) angeordnet ist;
optional umfasst die Indikatorlösung (110, 510) Öl oder eine wasserbasierte Lösung mit einem natürlich vorkommenden oder hinzugefügten Pigment, Farbadditiv oder Farbstoff.

7. Verfahren zum Messen und/oder Überwachen des Augeninnendrucks (IOP) eines Auges eines Subjekts, umfassend:
Anbringen einer flexiblen Linse (100, 500a, 500b, 500c) auf dem Auge, die flexible Linse bestehend aus einem oder mehreren hydrophilen sauerstoffdurchlässigen Polymeren, wobei die flexible Linse angepasst ist, um die Krümmung als Reaktion auf eine Änderung der Krümmung des Auges zu ändern;
die flexible Linse umfassend einen Mikrokanal (101, 501, 503), der Mikrokanal aufweisend mindestens zwei Enden, die zu einer Umgebung der Linse (100, 500a, 500b, 500c) offen sind, der Mikrokanal aufweisend eine Änderung des Volumens als Reaktion auf eine Änderung der Krümmung der Linse (100, 500a, 500b, 500c), und eine Indikatorlösung (110, 510), die in einem Abschnitt des Mikrokanals (101, 501, 503) mit Endpunkten (110a, 110b) der Indikatorlösung an einer Position zwischen den zwei oder mehr offenen Enden des Mikrokanals angeordnet ist;
Erfassen einer Änderung der Position der Endpunkte (110a, 110b) der Indikatorlösung (110, 510) in dem Mikrokanal (101, 501, 503); und
Korrelieren der Änderung der Position der Endpunkte (110a, 110b) der Indikatorlösung (110, 510) mit einer Änderung des IOP.

8. Verfahren nach Anspruch 7, der Mikrokanal (101, 501, 503) umfassend einen ersten Abschnitt, der linear oder in einem glatten Bogen eingerichtet ist, und einen zweiten Abschnitt, der in einer gewundenen, Zickzack-, gewickelten, spiralförmigen und/oder verzweigten Form entlang eines wesentlichen Abschnitts seiner Länge eingerichtet ist;
optional ist der Mikrokanal (101, 501, 503) eingerichtet, um einen äußeren Bereich der Linse einzunehmen.

9. Verfahren nach Anspruch 8, wobei die Indikatorlösung (110, 510) in dem ersten Abschnitt des Mikrokanals (101, 501, 503) angeordnet ist.

10. Verfahren nach Anspruch 7, umfassend das Erfassen von zwei oder mehr digitalen Bildern der Linse jeweils zu zwei oder mehr Zeitpunkten; und
Erfassen der Änderung der Position der Endpunkte (110a, 110b) der Indikatorlösung (110, 510) in dem Mikrokanal (101, 501, 503) als eine Differenz zwischen den zwei oder mehr digitalen Bildern;
optional werden die digitalen Bilder unter Verwendung einer Digitalkamera einer persönlichen Rechenvorrichtung oder unter Verwendung einer Digitalkamera, die angepasst ist, um von dem Subjekt getragen zu werden, erfasst;
optional ist die persönliche Rechenvorrichtung ein Smartphone.

11. Verfahren nach Anspruch 7, die flexible Linse (100, 500a, 500b, 500c) ferner umfassend mindestens einen Referenzmarker (106, 104, 108, 504);
wobei die Änderung der Position der Endpunkte (110a, 110b) der Indikatorlösung (110, 510) in dem Mikrokanal (101, 501, 503) relativ zu dem Referenzmarker (106, 104, 108, 504) bestimmt wird.

12. System zum Messen und/oder Überwachen des Augeninnendrucks (IOP) eines Auges eines Subjekts, umfassend:
die Vorrichtung nach Anspruch 1; und
mindestens eine Kamera;
wobei die mindestens eine Kamera angepasst ist, um von dem Subjekt getragen zu werden und digitale Bilder der Vorrichtung, die am Auge des Subjekts angebracht ist, zu erfassen;
optional ist die mindestens eine Kamera in einem Rahmen implementiert, der angepasst ist, um von dem Subjekt getragen zu werden;
optional ist der Rahmen ein Brillenrahmen.

13. System nach Anspruch 12, ferner umfassend eine Softwareanwendung, die angepasst ist, um das Erfassen von digitalen Bildern durch die mindestens eine Kamera zu steuern.

## Revendications

1. Dispositif de surveillance de la pression intraoculaire (IOP) d'un œil d'un sujet, comprenant :
une lentille flexible (100, 500a, 500b, 500c) constituée d'un ou de plusieurs polymères hydrophiles perméables à l'oxygène adaptée pour être placée sur l'œil et pour changer de courbure en réponse à un changement de courbure de l'œil ;
un microcanal (101, 501, 503) disposé dans ou sur la lentille, le microcanal ayant deux extrémités ou plus, dans lequel au moins deux extrémités sont ouvertes sur un environnement entourant la lentille (100, 500a, 500b, 500c) ;
une solution indicatrice (110, 510) disposée dans une partie du microcanal avec des points d'extrémité (110a, 110b) de la solution indicatrice à une position entre les deux extrémités ouvertes ou plus du microcanal (101, 501, 503) ;
dans lequel le microcanal présente un changement de volume en réponse à un changement de courbure de la lentille (100, 500a, 500b, 500c) ;
dans lequel le changement de volume du microcanal (101, 501, 503) entraîne un changement de la position des points d'extrémité (110a, 110b) de la solution indicatrice (110, 510) entre les deux extrémités ouvertes ou plus du microcanal (101, 501, 503) ;
dans lequel le changement de position des points d'extrémité (110a, 110b) de la solution indicatrice (110, 510) dans le microcanal (101, 501, 503) est indicatif d'un changement d'IOP.

2. Dispositif selon la revendication 1, comprenant :
au moins un marqueur de référence (106, 104, 108, 504) disposé sur la lentille (100, 500a, 500b, 500c) ;
dans lequel le changement de position des points d'extrémité (110a, 110b) de la solution indicatrice (110, 510) dans le microcanal (101, 501, 503) est déterminé par rapport à l'au moins un marqueur de référence (106, 104, 108, 504).

3. Dispositif selon la revendication 2, dans lequel ledit au moins un marqueur de référence (106, 104, 108, 504) et la solution indicatrice (110, 510) sont visibles dans des images numériques du dispositif.

4. Dispositif selon la revendication 1, comprenant un maillage, un bouchon poreux, ou une valve disposé dans ladite au moins une extrémité ouverte du microcanal (101, 501, 503).

5. Dispositif selon la revendication 1, dans lequel le microcanal (101, 501, 503) comprend une première section configurée linéairement ou en un arc lisse et une deuxième section configurée en une forme sinueuse, en zigzag, enroulée, en spirale, et/ou ramifiée le long d'une partie substantielle de sa longueur ;
facultativement le microcanal (101, 501, 503) est configuré pour occuper une région externe de la lentille.

6. Dispositif selon la revendication 5, dans lequel la solution indicatrice (110, 510) est disposée dans la première section du microcanal (101, 501, 503) ;
facultativement la solution indicatrice (110, 510) comprend de l'huile ou une solution à base d'eau avec un pigment naturel ou ajouté, un additif de couleur naturel ou ajouté, ou un colorant naturel ou ajouté.

7. Procédé de mesure et/ou de surveillance de la pression intraoculaire (IOP) d'un œil d'un sujet, comprenant :
adapter une lentille flexible (100, 500a, 500b, 500c) sur l'œil, la lentille flexible étant constituée d'un ou de plusieurs polymères hydrophiles perméables à l'oxygène, la lentille flexible étant adaptée pour changer de courbure en réponse à un changement de courbure de l'œil ;
dans lequel la lentille flexible comprend un microcanal (101, 501, 503), le microcanal ayant au moins deux extrémités qui sont ouvertes sur un environnement entourant la lentille (100, 500a, 500b, 500c), dans lequel le microcanal présente un changement de volume en réponse à un changement de courbure de la lentille (100, 500a, 500b, 500c), et une solution indicatrice (110, 510) disposée dans une partie du microcanal (101, 501, 503) avec des points d'extrémité (110a, 110b) de la solution indicatrice à une position entre les deux extrémités ouvertes ou plus du microcanal ;
détecter un changement de position des points d'extrémité (110a, 110b) de la solution indicatrice (110, 510) dans le microcanal (101, 501, 503) ; et
corréler le changement de position des points d'extrémité (110a, 110b) de la solution indicatrice (110, 510) avec un changement d'IOP.

8. Procédé selon la revendication 7, dans lequel le microcanal (101, 501, 503) comprend une première section configurée linéairement ou en un arc lisse et une deuxième section configurée en une forme sinueuse, en zigzag, enroulée, en spirale, et/ou ramifiée le long d'une partie substantielle de sa longueur ;
facultativement le microcanal (101, 501, 503) est configuré pour occuper une région externe de la lentille.

9. Procédé selon la revendication 8, dans lequel la solution indicatrice (110, 510) est disposée dans la première section du microcanal (101, 501, 503).

10. Procédé selon la revendication 7, comprenant :
capturer deux images numériques ou plus de la lentille à deux instants ou plus dans le temps, respectivement ; et
détecter le changement de position des points d'extrémité (110a, 110b) de la solution indicatrice (110, 510) dans le microcanal (101, 501, 503) en tant que différence entre les deux images numériques ou plus ;
facultativement les images numériques sont capturées en utilisant une caméra numérique d'un dispositif informatique personnel ou en utilisant une caméra numérique adaptée pour être portée par le sujet ;
facultativement le dispositif informatique personnel est un smartphone.

11. Procédé selon la revendication 7, dans lequel la lentille flexible (100, 500a, 500b, 500c) comprend en outre au moins un marqueur de référence (106, 104, 108, 504) ;
dans lequel le changement de position des points d'extrémité (110a, 110b) de la solution indicatrice (110, 510) dans le microcanal (101, 501, 503) est déterminé par rapport au marqueur de référence (106, 104, 108, 504).

12. Système de mesure et/ou de surveillance de la pression intraoculaire (IOP) d'un œil d'un sujet, comprenant :
le dispositif selon la revendication 1 ; et
au moins une caméra ;
dans lequel ladite au moins une caméra est adaptée pour être portée par le sujet et pour acquérir des images numériques du dispositif placé sur l'œil du sujet ;
facultativement ladite au moins une caméra est mise en œuvre dans un cadre qui est adapté pour être porté par le sujet ;
facultativement le cadre est un cadre de lunettes.

13. Système selon la revendication 12, comprenant en outre une application logicielle qui est adaptée pour commander l'acquisition d'image numérique par l'au moins une caméra.
